# EUROPÄISCHE PATENTSCHRIFT

(11) **EP 0 877 010 B1**
(45) Veröffentlichungstag und Bekanntmachung des Hinweises auf die Patenterteilung: **30.08.2000**
(21) Anmeldenummer: 98106109.6
(22) Anmeldetag: 03.04.1998
(51) Int. Cl.: C07C 19/08, A61K 47/06

(54) **Fluorierte Alkane und ihre Verwendungen**
Fluorinated alkanes and their uses
Alcanes fluorés et leurs utilisations

(30) Priorität: 07.05.1997 DE 19719280
(43) Veröffentlichungstag der Anmeldung: 11.11.1998
(73) Patentinhaber: PHARMPUR GmbH, 86156 Augsburg (DE)
(72) Erfinder: Menz, Dirk-Henning, Dr., 86420 Diedorf (DE)
(74) Vertreter: Rapp, Bertram, Dr.

(56) Entgegenhaltungen:
- EP-A- 0 444 752
- WO-A-93/01798
- WO-A-96/40052
- WO-A-97/12852
- DE-A- 19 536 504
- US-A- 5 275 669

## Beschreibung

Die Erfindung betrifft partiell fluorierte Alkane nach dem Oberbegriff des Anspruchs 1, also mit der Summenformel

**R**_{F} **[CF**₂ **- CH**₂**]R**_{H}

wobei R_{H} ein Substituent der allgemeinen Formel CₙHₓ ist und aus der Gruppe der n-Alkyle, s-Alkyle, t-Alkyle oder Cycloalkyle ausgewählt wird und R_{F} ein Substituent der allgemeinen Formel CₘFₓ ist und ausgewählt wird aus der Gruppe der Perfluor-n-Alkyle, Perfluor-s-Alkyle, Perfluor-t-Alkyle oder Perfluor-Cycloalkyle.

Falls s-Alkyle, t-Alkyle oder Cycloalkyle bzw. die perfluorierten Varianten dieser Alkyle verwendet werden, ist das Molekül verzweigt. Derartige, lineare bzw. verzweigte Moleküle sind beispielsweise in der DE 42 05 341 A1 und in der US 5,275,669 beschrieben. Aus diesen und weiteren Druckschriften ist ferner die Verwendung solcher Alkane in Medizin, Pharmazie und Technik bekannt. Insbesondere bei der DE 42 05 341 A 1 spielt die Reduzierung der Dichte eine entscheidende Rolle, was sich in der Wahl von Verbindungen mit kurzen Perfluorsubstituenten widerspiegelt. Fluorierte Alkane und ihre Verwendung beschreibt auch die DE 195 36 504 A1. Daneben gibt es eine Vielzahl weiterer Veröffentlichungen über fluorierte Alkane und deren Verwendungsmöglichkeiten, die jedoch aus Gründen der Übersichtlichkeit nicht alle aufgezählt werden. Die genannten Veröffentlichungen beschreiben den nach diesseitiger Kenntnis zur vorliegenden Erfindung nächstliegenden Stand der Technik.

Es ist darüberhinaus bekannt, daß durch Verlängerung von R_{F}- bzw. R_{H}-Substituenten und durch Kombination verschieden langer Substituenten in solchen Verbindungen Oberflächen- und Grenzflächeneigenschaften an Phasengrenzen zu anderen Medien bzw. von mit solchen Verbindungen behandelten Oberflächen beeinflußbar sind. Damit verbunden ist die Ausprägung hydrophiler bzw. hydrophober Eigenschaften, die sich in Löseeigenschaften bzw. lösungsvermittelnden Eigenschaften und in oberflächenaktiven Eigenschaften widerspiegeln.

Die aus dem Stand der Technik bekannten Variationen der R_{F}-bzw. R_{H}-Substituenten ergeben allerdings in der Regel keine Alkane mit optimalen biokompatiblen Eigenschaften, da die bei der Wechselwirkung mit anderen Stoffen entstehenden aggregierten Systeme häufig instabil sind. Hierdurch treten unerwünschte Entmischungen und geringe Lebensdauern der Mischungen und Emulsionen auf.

Unter Biokompatibilität ist in diesem Zusammenhang sowohl die chemische Inertheit eines Stoffes als auch dessen Gewebeverträglichkeit zu verstehen.

Die oben genannte Probleme der bekannten Substanzen treten auch in Anwendungen auf, bei denen die Biokompatibilität keine Rolle spielt. Unerwünschte Entmischungsvorgänge und geringe Lebensdauern der Substanzen sind in jedweder Anwendung von Nachteil, insbesondere bei der Verwendung dieser Substanzen als medizinische oder technische Lösungsmittel.

Es besteht daher die Aufgabe, ein fluoriertes Alkan so weiterzubilden, daß die sich bildenden aggregierten Systeme stabiler sind.

Gelöst wird diese Aufgabe mit den kennzeichnenden Merkmalen des Anspruchs 1. Vorteilhafte Verwendungsmöglichkeiten sind den Unteransprüchen entnehmbar.

Die erwünschten Eigenschaften werden dadurch erreicht, daß der Wasserstoffsubstituent R_{H} mindestens vier Kohlenstoffatome enthält und die Summe der Kohlenstoffatome im Wasserstoffsubstituenten R_{H} und im Fluorsubstituenten R_{F} kleiner als 18 ist sowie dadurch, daß mindestens einer der beiden Substituenten (Wasserstoffsubstituent R_{H} bzw. Fluorsubstituent R_{F}) als verzweigtes Alkyl ausgebildet ist.

Beispielsweise kann der Fluorsubstituent R_{F} ein Perfluor-n-Alkyl sein, während der Wassersubstituent R_{H} ein s-Alkyl, t-Alkyl oder ein Cycloalkyl sein kann. Alternativ hierzu kann der Wasserstoffsubstituent R_{H} ein n-Alkyl sein, wobei dann der Fluorsubstituent verzweigt ist, also ein Perfluor-s-Alkyl oder ein Perfluor-t-Alkyl oder ein Perfluor-Cycloalkyl ist. Auch beide Substituenten R_{F} und R_{H} können verzweigt sein, allerdings ist es erfindungsgemäß ausgeschlossen, daß beide Substituenten linear sind.

Die Synthese der genannten Verbindungen ist für den Fachmann mit den fachnotorisch üblichen Methoden ohne weiteres durchzuführen und wird daher nicht näher beschrieben.

Die beschriebenen Verbindungen werden insbesondere vor medizinischen Anwendungen hochgereinigt, und zwar mit Hilfe an sich bekannter Verfahren. Die so hergestellten Verbindungen sind dann völlig untoxisch, da weder an der Spacergruppe CF₂ - CH₂ noch am perfluorierten Teil der Verbindung Austauschreaktionen stattfinden können, die zu toxischen Wirkungen führen würden. Die besonderen Lösemitteleigenschaften der Verbindungen beruhen darauf, daß sie nicht nur die Eigenschaften reiner Perfluorcarbone mit den Eigenschaften reiner Kohlenwasserstoffe in sich vereinigen, sondern nach dem Lösevorgang aggregierte Systeme verschiedenster Struktur bilden, die durch die anisotrope Strukur der erfindungsgemäßen Verbindungen stabilisiert werden. Dies wird ermöglicht durch die erfindungsgemäße Wahl der Substituenten, wobei auch der unterschiedliche Raumbedarf der R_{F}- und R_{H}-Substituenten eine Rolle spielt. Die strukturbedingte Stabilisierung der aggregierten Systeme unterdrückt die Entmischung der entstehenden Lösungen und erhöht die Lösekraft der Verbindungen. Vergleichbare Effekte können in rein linearen R_{F}-R_{H} Verbindungen nur durch wesentlich größere Kettenlängen bzw. Kettenlängendifferenzen erzielt werden, was gleichzeitig zu unerwünschten Eigenschaften wie beispielsweise höheren Siedepunkten bzw. wachsartigen Verbindungen führt, wodurch ein Einsatz als biokompatibles Lösungsmittel und flüssiges Implantat in der Regel nicht mehr möglich ist und auch die anderen Verwendungen nur eingeschränkt möglich sind.

Das erfindungsgemäße Alkan kann besonders in folgenden Anwendungsfällen vorteilhaft verwendet werden.

Durch das Lösevermögen für Kohlenwasserstoffe, insbesondere Mineralöle, Teer und Siliconöle, ermöglichen die erfindungsgemäßen Alkane die Reinigung von mit solchen Stoffen kontaminierten Geweben. Die Verunreinigungen bilden mit den erfindungsgemäßen Alkanen stabile Lösungen, so daß sie ausgewaschen werden können. Auf diese Weise können Siliconöle, die aus defekten Implantatkissen ausgetreten sind bzw. als Flüssigimplantate in der Augenheilkunde genutzt werden, schonend entfernt werden. Besonders eignen sich die erfindungsgemäßen Alkane für die Versorgung von Verletzungen, in die Öl, Teer oder Fette auf Mineralölbasis eingedrungen sind. Insbesondere wird auch die Entfernung von Verbrennungsrückständen aus Brandverletzungen möglich. Durch die Fähigkeit der erfindungsgemäßen Alkane, Sauerstoff zu transportieren, wird die Dekontamination der Gewebe durch die Versorgung mit Sauerstoff zusätzlich unterstützt.

Ein wesentlicher Unterschied der erfindungsgemäßen Alkane zu herkömmlichen Lösungsmitteln besteht darin, daß herkömmliche Lösungsmittel die zu behandelnden Gewebe nachhaltig schädigen, nicht dagegen die erfindungsgemäßen Alkane.

Im weiteren können die erfindungsgemäßen Alkane als Hilfsmittel zur Regeneration von Gewebe in Raucherlungen eingesetzt werden, indem die Ablagerungen innerhalb der Lunge durch Spülungen der Lunge ausgewaschen werden. Durch das hohe Lösevermögen der erfindungsgemäßen Alkane für Sauerstoff und Kohlendioxid kann die Sauerstoffversorgung aufrechterhalten werden und der Abtransport von Kohlendioxid wird nicht unterbrochen.

In ihrer Eigenschaft als Lösungsmittel weisen die erfindungsgemäßen Alkane den Vorteil auf, daß Größe und räumliche Struktur der R_{F} und R_{H} Substituenten derart geeignet gewählt werden können, daß das Herauslösen von Verunreinigungen unter minimaler Gewebepenetration erfolgen kann.

Die biokompatiblen Eigenschaften und die geringe Oberflächenspannung sowie die besonderen Grenzflächeneigenschaften der Alkane ermöglichen es, diese Verbindungen als flüssige Implantate zu nutzen. Ein Beispiel dafür ist die Verwendung in der Augenheilkunde, z.B. als Glaskörpersubstitut, als Netzhautentfaltungsmittel, als Hilfsmittel bei der Laserkoagulation der Netzhaut oder als postoperative Netzhauttamponade.

Falls im Bereich der Augenheilkunde oder der Wundversorgung unerwünschte Substanzen, z.B. Siliconöl, Teer oder Fette ausgewaschen werden sollen, müssen häufig sehr wirksame Lösungsmittel eingesetzt werden, welche aber auch das Gewebe nachhaltig schädigen können. Die erfindungsgemäßen Alkane können hier durch ein Zwei-Schritt-Verfahren sinnvoll eingesetzt werden. In einem ersten Schritt wird das Siliconöl, der Teer oder das Fett mit einem wirksamen, aber eventuell gewebeschädlichen Lösungsmittel ausgespült und in einem zweiten, darauffolgenden Schritt wird dieses Lösungsmittel mit einem der erfindungsgemäßen Alkane ausgewaschen.

Das beschriebene Verfahren kann auch in mehreren Schritten ausgeführt werden, wobei durch schrittweisen Austausch der Lösungsmittel einzelne Wirkungen dieser Lösungsmittel zeitlich begrenzt genutzt und somit optimal den therapeutischen Erfordernissen angepaßt werden können. Bei diesen Verfahren können einige oder alle angewandten Lösungsmittel unter die erfindungsgemäße Substanzklasse fallen.

In ähnlicher Weise können die erfindungsgemäßen Alkane mit Hilfe eines hochwirksamen Lösungsmittels als flüssiges Skalpell eingesetzt werden. Hierzu wird zunächst ein Lösungsmittel, das bewußt Gewebebestandteile angreift, eingebracht und danach durch Zugabe eines der erfindungsgemäßen "Alkane entschärft". Dies entspricht einer chemischen Alternative zu den im biologischen Bereich bekannten Wirkungen von Entzymen, die bereits für solche spezifischen Abbaureaktionen genutzt werden.

Durch den amphiphilen Charakter der Verbindungen werden weitere Anwendungsgebiete erschlossen. Die erfindungsgemäßen Alkane können mit pharmakologischen Wirkstoffen Mikroemulsionen bilden, in denen die Wirkstoffe von den Alkanen eingeschlossen sind. Auf diese Weise können sogenannte "slow-drug-release"-Systeme aufgebaut werden, also Substanzen, die bestimmte Pharmaka umschließen und diese erst langsam freigeben.

Eine weitere Nutzung des amphiphilen Charakters der Verbindungen ist die Stabilisierung sauerstofftransportierender Medien, die aus Emulsionen perfluorierter Verbindungen aufgebaut sind. Sauerstofftransportierende Medien finden insbesondere als Blutersatzstoffe oder bei der Flüssigbeatmung eine Rolle.

Die erfindungsgemäßen Alkane können darüberhinaus wegen ihrer besonderen Lösungsmitteleigenschaften auch in der Technik eingesetzt werden, nämlich als Reinigungs- und/oder Lösungsmittel. Dies ist insbesondere vorteilhaft, wenn eine direkte Berührung des Menschen mit dem Lösungsmittel nicht vermeidbar ist oder aber Reste von Lösungsmittel auf oder in den mit diesen Lösungsmitteln behandelten technischen bzw. medizintechnischen Produkten verbleiben und nicht völlig entfernt werden können und auf diesem Wege in den menschlichen Körper gelangen. Dazu zählt die Reinigung von Implantaten und Prothesen ebenso wie das Auftragen dünner Siliconölfilme auf Kanülen und anderen medizintechnischen Einrichtungen, um deren Gleitfähigkeit zu erhöhen.

Ferner können die erfindungsgemäßen Alkane selbst als Gleitmittel eingesetzt werden, indem beispielsweise Kunststoffoberflächen mit diesen Verbindungen eingerieben werden. Dabei richten sich die Alkane in der Weise aus, daß der R_{H} Substituent sich zur Kunststoffoberfläche hin orientiert und die dann nach außen gerichteten fluorierten Bereiche die Oberflächen in bekannter Weise modifizieren.

In der Verwendung als Lösungsmittel für Kohlenwasserstoffe und Siliconöle haben sich Alkane nach der eingangs genannten Formel als besonders vorteilhaft erwiesen, bei n > 7 ist und m einen Wert zwischen 5 und 8 besitzt.

In der Verwendung als flüssiges Implantat und als Implantat mit tamponierender Wirkung sind Alkane nach der eingangs genannten Formel besonders vorteilhaft, wenn m > 5 ist und n Werte zwischen m - 1 und m + 2 annimmt.

In der Verwendung als Amphiphil sind Alkane der eingangs genannten Formel besonders vorteilhaft, wenn das Verhältnis des sterischen Volumens der Substituenten R_{F} zu R_{H} kleiner als 0,7 bzw. größer als 1,3 ist.

Wie oben beschrieben, muß die Synthese der erfindungsgemäßen Alkane nicht näher dargelegt werden, da sie für den Fachmann ohne weiteres möglich ist. Beispielsweise kann das Alkan

R_{F(4)} [CF₂ - CH₂ ] R_{H(3)}

anders geschrieben 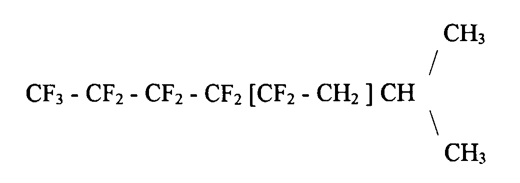 welches seinen Siedepunkt bei 143° C hat, hergestellt werden durch Entwässern von 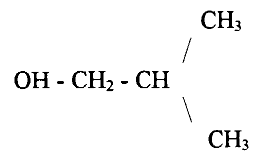 anschließende Addition von C₅F₁₁I, Hydrierung mit Zn/HCl und Destillation sowie anschließende Hochreinigung.

Im folgenden werden einige weitere Herstellungsbeispiele angegeben.

### 1-Perfluorbutyl-2-methyl-propan (CF₃-CF₂-CF₂-[CF₂-CH₂ ]-CH-(CH₃ )₂ )

Perfluorbutyljodid wird zusammen mit AIBN (Azo-bis-isobutyronitril) als Radikalstarter im Autoklaven unter Kühlung 30 min mit Stickstoff gespült und anschließend Isobuten im molaren Verhältnis einkondensiert. Anschließend wird im geschlossenem System unter Rühren auf 90°C erhitzt und bei dieser Temperatur ca. 6 h weitergerührt.
Nach der destillativen Abtrennung von unumgesetzten Ausgangsstoff (Vigreuxkolonne, verminderter Druck) wird mit Zn/HCl hydrierend dehalogeniert und das Produkt im Vakuum über eine Vigreuxkolonne fraktioniert destilliert. Kp.: 70°/60 Torr., Ausbeute ca. 40 % (GC). Das Produkt wird anschließend nach bekanntem Verfahren hochgereinigt.

### 1-Perfluorhexyl-2-methyl-propan (CF₃-CF₂-CF₂-CF₂-CF₂-[CF₂-CH₂ ]-CH-(CH₃ )₂ )

Perfluorhexyljodid wird zusammen mit AIBN als Radikalstarter im Autoklaven unter Kühlung 30 min mit Stickstoff gespült und anschließend Isobuten im molaren Verhälntis einkondensiert. Anschließend wird im geschlossenem System unter Rühren auf 90°C erhitzt und bei dieser Temperatur ca. 6 h weitergerührt.
Nach der destillativen Abtrennung von unumgesetzten Ausgangstoff (Vigreuxkolonne, verminderter Druck) wird mit Zn/HCl hydrierend dehalogeniert und das Produkt im Vakuum über eine Vigreuxkolonne fraktioniert destilliert. Kp.: 80°/60 Torr.; Ausbeute ca. 40 % (GC). Das Produkt wird anschließend nach bekanntem Verfahren hochgereinigt.

### 1-Perfluorbutyl-3-methyl-butan (CF₃-CF₂-CF₂-[CF₂-CH₂ ]-CH₂-CH-(CH₃ )₂ )

Isoamylalkohol wird mit 85 %iger Phosphorsäure in das entsprechende Penten überführt, destillativ gereinigt und im molaren Verhältnis mit Perfluorbutyljodid in einen Autoklaven gegeben. Nach Zugabe von AIBN wird unter Kühlung 30 min mit N₂ gespült und anschließend wie im Beispiel 1 fortgefahren. Ausbeute ca. 35 % (GC), Kp.: 65°/60 Torr.

### 1-Perfluorhexyl-3-methyl-butan (CF₃-CF₂-CF₂-CF₂-CF₂-[CF₂-CH₂ ]-CH(CH₃)-CH₂CH₃ )

Isoamylalkohol (Isomere) wird mit konzentrierter Schwefelsäure in das entsprechende Penten überführt, destallativ gereinigt und im molaren Verhältnis mit Perfluorhexyljodid in einen Autoklaven gegeben. Nach Zugabe von AIBN wird unter Kühlung 30 min mit N₂ gespült und anschließend wie im Beispiel 1 fortgefahren. Ausbeute ca. 35 % (GC), Kp.: 85°/60 Torr.

## Patentansprüche

1. Teilfluoriertes Alkan mit der Summenformel
**R**_{F} **[CF**₂ **- CH**₂**]R**_{H}
wobei R_{H} ein Substituent der allgemeinen Formel CₙHₓ ist und ausgewählt ist aus der Gruppe der n-Alkyle, s-Alkyle, t-Alkyle oder Cycloalkyle und R_{F} ein Substituent der allgemeinen Formel CₘFₓ ist und ausgewält ist aus der Gruppe der Perfluor-n-Alkyle, Perfluor-s-Alkyle, Perfluor-t-Alkyle oder Perfluor-Cycloalkyle, **dadurch gekennzeichnet**, daß n ≥ 3 ist, die Summe von n + m < 18 ist und nur entweder R_{H} oder R_{F} ein n-Alkyl bzw. Perfluor-n-Alkyl sind.

2. Alkan nach Anspruch 1, **dadurch gekennzeichnet**, daß n > 7 ist und m zwischen 5 und 8 liegt.

3. Alkan nach Anspruch 1, **dadurch gekennzeichnet**, daß m > 5 ist und n zwischen m - 1 und m + 2 ist.

4. Alkan nach Anspruch 1, **dadurch gekennzeichnet**, daß das Verhältnis des sterischen Volumens von R_{F} zu R_{H} kleiner als 0,7 ist.

5. Alkan nach Anspruch 1, **dadurch gekennzeichnet**, daß das Verhältnis des sterischen Volumens von R_{F} zu R_{H} größer als 1,3 ist.

6. Verwendung eines Alkans nach einem der Ansprüche 1 bis 5 als biokompatibles Lösungsmittel, insbesondere für Perfluorcarbone und Kohlenwasserstoffe.

7. Verwendung eines Alkans nach einem der Ansprüche 1 bis 5 als Bestandteil eines sauerstofftransportierenden Mediums, insbesondere eines Blutersatzstoffes.

8. Verwendung eines Alkans nach einem der Ansprüche 1 bis 5 als Bestandteil eines flüssigen Implantats.

9. Verwendung eines Alkans nach einem der Ansprüche 1 bis 5 als Lösungsmittel oder Lösungsvermittler für Pharmaka.

10. Verwendung eines Alkans nach einem der Ansprüche 1 bis 5 als technisches Reinigungsmittel.

11. Verwendung eines Alkans nach einem der Ansprüche 1 bis 5 als Additiv zu mindestens einem bekannten Perfluorcarbon.

12. Verwendung eines Alkans nach einem der Ansprüche 1 bis 5 als Lösungsmittel für Beschichtungsmaterialien für medizinische Geräte zur Erhöhung deren Gleitfähigkeit.

13. Verwendung eines Alkans nach einem der Ansprüche 1 bis 5 als medizinisches Hilfsmittel, insbesondere in der Augenheilkunde als Glaskörpersubstitut oder Netzhautentfaltungsmittel oder Hilfsmittel bei der Laserkoagulation an der Netzhaut oder Lösungsmittel für Arzneimittel oder postoperative Netzhauttamponade.

14. Verwendung eines Alkans nach einem der Ansprüche 1 bis 5 als medizinisches Hilfsmittel in der Dermatologie.

15. Verwendung eines Alkans nach einem der Ansprüche 1 bis 5 als technisches Gleitmittel bzw. Gleitmitteladditiv.

16. Verwendung eines Alkans nach einem der Ansprüche 1 bis 5 als medizinisches Hilfsmittel bei der Wundversorgung, insbesondere von Brandwunden zur Reinigung von Verbrennungsrückständen.

17. Verwendung eines Alkans nach einem der Ansprüche 1 bis 5 als medizinisches Hilfsmittel zur Regenerierung von Lungengewebe durch Spülen der Lunge.

## Claims

1. Partially fluorinated alkane with the total formula
R_{F} [CF₂ - CH₂]R_{H}
in which R_{H} is a substituent of the general formula CₙHₓ and is selected from the group of n-alkyls, s-alkyls, t-alkyls or cycloalkyls and R_{F} is a substituent of the general formula CₘFₓ and is selected from the group of the perfluoro-n-alkyls, perfluoro-s-alkyls, perfluoro-t-alkyls or perfluoro-cycloalkyls, characterised in that n is ≥ 3, the sum of n + m is < 18 and only either R_{H} or R_{F} is an n-alkyl or perfluoro-n-alkyl.

2. Alkane according to claim 1, characterised in that n is > 7 and m lies between 5 and 8.

3. Alkane according to claim 1, characterised in that m is > 5 and n lies between m-1 and m+2.

4. Alkane according to claim 1, characterised in that the ratio of the steric volume of R_{F} to R_{H} is less than 0.7.

5. Alkane according to claim 1, characterised in that the ratio of the steric volume of R_{F} to R_{H} is greater than 1.3.

6. Use of an alkane according to one of claims 1 to 5 as a bio-compatible solvent, in particular for perfluorocarbons and hydrocarbons.

7. Use of an alkane according to one of claims 1 to 5 as a component of an oxygen-transporting medium, in particular a blood substitute.

8. Use of an alkane according to one of claims 1 to 5 as a component of a liquid implant.

9. Use of an alkane according to one of claims 1 to 5 as a solvent or solubilizer for drugs.

10. Use of an alkane according to one of claims 1 to 5 as a technical cleaning agent.

11. Use of an alkane according to one of claims 1 to 5 as an additive for at least one known perfluorocarbon.

12. Use of an alkane according to one of claims 1 to 5 as a solvent for coating materials for medical equipment for increasing their sliding ability.

13. Use of an alkane according to one of claims 1 to 5 as a medical accessory, in particular in ophthalmology as a vitreous humor replacement or retinal smoothing agent or accessory in laser coagulation on the retina or solvent for drugs for post-operative retina tamponade.

14. Use of an alkane according to one of claims 1 to 5 as a medical accessory in dermatology.

15. Use of an alkane according to one of claims 1 to 5 as a technical lubricant or lubricant additive.

16. Use of an alkane according to one of claims 1 to 5 as a medical accessory for treatment of wounds, in particular burns for cleaning burn residues.

17. Use of an alkane according to one of claims 1 to 5 as a medical accessory for regenerating lung tissue by lavage of the lung.

## Revendications

1. Alcane partiellement fluoré ayant pour formule brute
R_{F}[CF₂-CH₂]R_{H}
dans laquelle R_{H} est un substituant de la formule générale CₙHₓ et choisi dans le groupe des n-alkyles, s-alkyles, t-alkyles ou cycloalkyles, et R_{F} est un substituant de la formule générale CₘFₓ et choisi dans le groupe des perfluoro-n-alkyles, perfluoro-s-alkyles, perfluoro-t-alkyles ou perfluorocycloalkyles, **caractérisé en ce que** n est ≥ 3, la somme de n + m est < 18 et soit R_{H}, soit R_{F} seulement sont un n-alkyle ou perfluoro-n-alkyle.

2. Alcane selon la revendication 1, **caractérisé en ce que** n est > 7 et m est compris entre 5 et 8.

3. Alcane selon la revendication 1, **caractérisé en ce que** m est > 5 et n est compris entre m - 1 et m + 2.

4. Alcane selon la revendication 1, **caractérisé en ce que** le rapport du volume stérique de R_{F} sur R_{H} est inférieur à 0,7.

5. Alcane selon la revendication 1, **caractérisé en ce que** le rapport du volume stérique de R_{F} sur R_{H} est supérieur à 1,3.

6. Utilisation d'un alcane selon l'une des revendications 1 à 5 comme solvant biocompatible, en particulier pour des perfluorocarbones et des hydrocarbures.

7. Utilisation d'un alcane selon l'une des revendications 1 à 5 comme constituant d'un milieu transportant l'oxygène, en particulier d'un substitut du sang.

8. Utilisation d'un alcane selon l'une des revendications 1 à 5 comme constituant d'un implant liquide.

9. Utilisation d'un alcane selon l'une des revendications 1 à 5 comme solvant ou agent de solubilisation pour des produits pharmaceutiques.

10. Utilisation d'un alcane selon l'une des revendications 1 à 5 comme agent nettoyant technique.

11. Utilisation d'un alcane selon l'une des revendications 1 à 5 comme additif à au moins un perfluorocarbone connu.

12. Utilisation d'un alcane selon l'une . des revendications 1 à 5 comme solvant pour des matériaux de revêtement d'instruments médicaux afin d'augmenter l'aptitude au glissement de ces derniers.

13. Utilisation d'un alcane selon l'une des revendications 1 à 5 comme adjuvant médical, en particulier en ophtalmologie comme substitut du corps vitré ou comme agent d'épanouissement de la rétine, ou bien comme adjuvant pour la coagulation rétinienne au laser, ou bien comme solvant pour des médicaments ou des tamponnements rétiniens post-opératoires.

14. Utilisation d'un alcane selon l'une des revendications 1 à 5 comme adjuvant médical en dermatologie.

15. Utilisation d'un alcane selon l'une des revendications 1 à 5 comme agent de glissement technique ou comme additif pour agent de glissement.

16. Utilisation d'un alcane selon l'une des revendications 1 à 5 comme adjuvant médical pour soigner les plaies, notamment les brûlures afin de nettoyer les résidus de tissus brûlés.

17. Utilisation d'un alcane selon l'une des revendications 1 à 5 comme adjuvant médical pour régénérer les tissus pulmonaires par lavage des poumons.
